Europäisches Patentamt

European Patent Office

Office européen des brevets

(19)

(11) Publication number: **0 281 069**
A1

(12) **EUROPEAN PATENT APPLICATION**

(21) Application number: **88103067.0**

(22) Date of filing: **01.03.88**

(51) Int. Cl.4: **C12N 15/00** , C12P 21/00 ,
C12N 5/00 , C12N 1/20 ,
//(C12N1/20,C12R1:19)

(30) Priority: **02.03.87 JP 48580/87**

(43) Date of publication of application:
**07.09.88 Bulletin 88/36**

(84) Designated Contracting States:
**CH DE FR GB LI**

(71) Applicant: **SUMITOMO CHEMICAL COMPANY, LIMITED**
**15 Kitahama 5-chome Higashi-ku**
**Osaka-shi Osaka 541(JP)**

Applicant: **SUMITOMO PHARMACEUTICALS COMPANY, LIMITED**
**40, Dosho-machi 2-chome**
**Higashi-ku Osaka-shi Osaka-fu(JP)**

(72) Inventor: **Nakayama, Chikao**
**5-1-123, Honmachi 9-chome**
**Toyonaka-shi Osaka-fu(JP)**
Inventor: **Okamoto, Minoru**
**15-10-204, Kusunoki-cho**
**Ashiya-shi Hyogo-ken(JP)**
Inventor: **Yanagi, Hideki**
**14-26, Matsugaoka Hanayashiki**
**Takarazuka-shi Hyogo-ken(JP)**

(74) Representative: **Vossius & Partner**
**Siebertstrasse 4 P.O. Box 86 07 67**
**D-8000 München 86(DE)**

(54) Production of human granulocyte-macrophage colony stimulating factor.

(57) Human granulocyte-macrophage colony stimulating factor produced in human cells transfected with an exogenous DNA encoding said factor.

EP 0 281 069 A1

# PRODUCTION OF HUMAN GRANULOCYTE-MACROPHAGE COLONY STIMULATING FACTOR

The present invention relates to human granulocyte-macrophage colony stimulating factor produced in human cells transfected with DNA encoding the said factor and to a process for producing the said factor which comprises culturing the human cells transfected with DNA coding for the said factor and recovering it.

Granulocyte-macrophage colony stimulating factor (hereinafter referred to as "GM-CSF") is a member of the family of different types of colony stimulating factors, which are known to have an ability to stimulate the differentiation of progenitor cells in bone marrow into mature blood cells. GM-CSF stimulates production of granulocytes and macrophages from the corresponding progenitor cells and has an in vitro activity to stimulate the myeloid progenitor cells to form colonies of granulocytes and macrophages (A.W. Burgess et al., J.Biol.Chem., 252(1977): 1998-2003).

It has been known that the human granulocyte-macrophage colony stimulating factor (hereinafter referred to as "hGM-CSF") is useful for the treatment of infectious diseases or leukocytepenia which are caused by, for example, irradiation therapy or chemotherapy of cancer patients.

It is known that, for a large scale production of substances naturally occurring in small amounts such as hGM-CSF, recombinant DNA technology is one of the most practical and advantageous methods.

The isolation of cDNA clones coding for human GM-CSF and the expression of hGM-CSF in E.coli and animal cells have already been reported by G.Wong et al., and F.Lee et al. (Science, 228:810 (1985); International Patent Publication No. WO86/639; Proc.Nat.Acad.Sci., 82:4360 (1985); International Patent Publication No. WO86/3225).

In the above mentioned methods, the cloned DNA encoding hGM-CSF ligated downstream of the promoter of simian virus 40 (SV40) containing an origin of replication (ori) was introduced into monkey kidney COS cells and the resulting transformant COS cells were cultured to express human GM-CSF.

It is to be noted, however, that the expression of hGM-CSF in the above methods was only transient due to the fact that the used plasmids containing the DNA encoding hGM-CSF were not integrated into the chromosomes of the host cells, but autonomously replicated in the host cells to several tens of thousands of copies to kill the host cells. Thus, the above mentioned methods are not a commercially advantageous method for the large-scale production of hGM-CSF.

As host cells, Chinese hamster ovary (CHO) cells defective in dihydrofolate reductase (DHFR) were also employed to express hGM-CSF. The use of DHFR deficient CHO cells has advantages such as that the level of expression of the inserted gene in DHFR deficient CHO cells can be increased by treatment with the DHFR-inhibitor methotrexate (MTX), and that the expression of the inserted gene permanently continues in the host cells. However, CHO cells have the disadvantage that they are adherent cells, which causes various technical difficulties in large-scale cultivation of the cells.

Although CHO cells have an ability to add sugar chains to polypeptides, it is unlikely that the sugar chain structures of the expression products of inserted human genes are identical to those of the naturally-occurring human substances. It has been reported that the sugar chain structure of the erythropoietin is different from that of the naturally-occuring erythropoietin when produced in CHO cells (EP-A2-148 605 corresponding to International Patent Publication No.WO85/2610).

In the case of glycoproteins to be used as therapeutic agents, particularly those to be injected to humans, their sugar chain structures are very important because administration of glycoproteins with non-human-type sugar chain structure may cause undesirable antigenicity to the recipients.

In order to solve these problems, an extensive study was done on the production of human GM-CSF by recombinant DNA technology and the present inventors have succeeded for the first time in expression of the DNA encoding human GM-CSF in human host cells and purification of the resulting human GM-CSF.

In the process of the present invention, human GM-CSF is efficiently prepared by culturing human cells, particularly Namalwa cells, which have been transfected with plasmids containing the DNA sequence coding for hGM-CSF and recovering the resulting hGM-CSF in highly purified forms. The use of human cells as a host in the production of human GM-CSF provides very important advantages in that the sugar chains of the product, human GM-CSF are quite similar or identical to those of natural human GM-CSF. Additionally, Namalwa cells have desirable characteristics as a host cell to express human GM-CSF in that they are non adherent cells and can be efficiently grown in suspension culture to produce human GM-CSF.

## SUMMARY OF THE INVENTION

The present invention provides a process for producing human granulocyte-macrophage colony stimulating factor which comprises culturing human cells which have been transfected with plasmids containing the DNA sequence coding for human granulocyte-macrophage colony stimulating factor in a suitable medium and recovering the resulting human granulocyte-macrophage colony stimulating factor. It also provides a transformed human cell which is produced by transfection of a human cell with the said plasmids. It further provides recombinant human granulocyte-macrophage colony stimulating factor produced in the said transformed human cells.

In the present invention, the hGM-CSF gene to be used for the transfection of host cells may be either cDNA or genomic DNA coding for hGM-CSF. The genomic DNA may be isolated from a human genomic library such as the library of Maniatis (Cell, 15:1157 (1978)). The hGM-CSF cDNA clones may be prepared from the cells efficiently producing hGM-CSF such as human peripheral blood monocytes, urinary bladder tissue, placental tissue or T-cell lymphoma by conventional methods.

The isolated genomic DNA or cDNA encoding hGM-CSF is then built up to be expressed in human cells. For example, to the 5' end of the isolated cDNA coding for hGM-CSF, one or more promoters functional in higher eukaryote cells such as the promoter of the human GM-CSF gene or promoters of animal viruses (e.g., SV40) are connected, and to the 3' end a polyadenylation site and an intron derived from, for example, the human GM-CSF gene, SV40 early gene or $\beta$-globin gene so that the gene are efficiently transcribed and translated in the host cells.

Enhancer sequences effective in human lymphocytes may be ligated in the vicinity of the DNA coding for human GM-CSF to enhance the transcription level. Examples of such enhancers are the enhancer of SV40 or adenovirus or retroviruses or immunoglobulin gene. For this purpose, an expression vector pKCR may be used as shown in the following example. The expression vector pKCR can be constructed by the method disclosed by K.O'Hare et al. (Pro.Nat.Acad.Sci., USA, 78:1527 (1981)).

For facilitation of selection of transformants, selection makers such as neomycin resistance gene or xanthine-guanine phosphoribosyl transferase gene may be preferably introduced in the host cells together with the DNA coding for the human GM-CSF.

The following methods are used for the introduction of the DNA encoding hGM-CSF into the host cells: .

1) a method in which DNA is introduced into host cells as a complex with calcium phosphate (F.Graham et al., Virology, 52:456 (1973));

2) a method in which DNA is combined with DEAE-dextran to introduce the DNA into host cells (L.Sompayrac et al., Pro.Nat.Acad.Sci., USA, 78:7575 (1981));

3) a method in which protoplasts of E.coli harboring the plasmids coding for a desired DNA are fused with the host cells (V.T.Oi et al., Pro.Nat.Acad.Sci., USA, 80:825 (1983));

4) a method of introducing DNA into host cells by electroporation (H.Potter et al., Pro.Nat.Acad.Sci., USA, 81:7161 (1984)).

The transformants which have acquired an ability to produce hGM-CSF are prepared as described above and they may be grown in a culture medium usually used for cultivation of animal cells. For example, they may be cultured in RPMI1640 supplemented with 10% fetal bovine serum at about 37°C under an atmosphere of saturated water vapor containing 5% $CO_2$. The cultivation of the transformants may be conducted in various fashions. They may be cultured in flasks or dishes or grown in spinner vessels. They may also be grown in hollow fiber equipments while continuously circulating a culture medium or in jar fermentors for animal cell culture.

The hGM-CSF secreted in the culture medium can be recovered and purified by a column chromatography on anti-hGM-CSF-linked water-insoluble matrixes after removing the cells by centrifugation or filtration.

The methods used for the isolation of GM-CSF from the media of hGM-CSF-producing Mo lymphocytes or COS cells transfected with hGM-CSF expression vectors can also be used for the isolation of the hGM-CSF produced by the process of the present invention (G.G.Wong et al., Science, 228:810 (1985); International Patent Publication No. WO86/00639)). The hGM-CSF produced by the process of the present invention has been found to have the following amino terminal sequence:

N-terminal: Ala-Pro-Ala-Arg-Ser-

SDS polyacrylamide gel electrophoresis revealed that the hGM-CSF migrated as a broad band with apparent molecular weights of 22,000 to 36,000. It is known that there are two asparagine-linked sugar chains and two mucin-type sugar chains in hGM-CSF (R.E.Donahue et al., Cold Spring Harbor Symposia on Quantitative Biology, 51:685 (1986)).

Not only the naturally-occurring hGM-CSF produced in peripheral blood lymphocytes or Mo cells but also the recombinant hGM-CSF produced in CHO cells are mixture of the hGM-CSFs attaching asparagine-linked sugar chains at both sites (2N type), the ones attaching the sugar chain at only one site (1N type) and the ones having only mucin-type sugar chains (0N type). On SDS polyacrylamide gel electrophoresis, the 2N type hGM-CSF migrates as a broad band centering around the position of an apparent molecular weight of 30,000, 1N type around 22,000 and 0N type around 14,000. The SDS polyacrylamide gel electrophoresis analy sis of the hGM-CSF produced in the present invention indicates that the hGM-CSF of the present invention, which have 22,000 - 36,000 of apparent molecular weights, is 2N type.

The broadness of the apparent molecular weight of various type hGM-CSF, 2N, 1N or 0N types is due to the microheterogeneity of the sugar chain structures as is generally observed in other kinds of glycoproteins.

## BRIEF DESCRIPTION OF THE DRAWINGS

Fig. 1 is a diagram indicating the restriction sites in the mouse GM-CSF cDNA used as a probe in the following Example 1.

The dotted line indicates the region used as the probe to screen hGM-CSF cDNA and the number indicates the nucleotide number counted from the 5′ end of the cDNA.

Fig. 2 depicts the DNA sequence and the deduced amino acid sequence for hGM-CSF derived from human cell line U937.

The underlined amino acid, alanine, indicates the N-terminal residue of the mature hGM-CSF.

The double underline indicates the amino acid which is different from the corresponding amino acid residue of the hGM-CSF derived from Mo cells.

Fig. 3 is a diagram indicating the construction of the plasmid pCGMU.

Fig. 4 is a diagram indicating the construction of the plasmid pCGMUdhfr.

Fig. 5 represents the number of the colonies of KG-1 cells formed in culturing the cells in the presence of the supernatant of the culture media of the Namalwa cells transfected with the plasmid pCGMU or pKCR. The number on the vertical line indicates the number of the KG-1 cell colonies and the horizontal line indicates the rate of dilution of the supernatant.

The open circles indicate the experiments with the Namalwa cells transfected with the plasmid pCGMU and solid circles indicate those with pKCR.

Fig. 6 represents the enhancement of the survival of human neutrophils cultured in the presence of the supernatant of the culture media of the Namalwa cells transfected with the plasmid pCGMU and pKCR. The number on the horizontal line indicates the rate of dilution of the added supernatant.

Fig. 7 represents the number of the colonies of KG-1 cells formed in culturing the cells in the presence of the supernatant of the culture media of the Namalwa F11 and the wild-type Namalwa cells. The number on the vertical line indicates the number of the KG-1 cell colonies and the horizontal line indicates the rate of dilution of the supernatant. The open circles indicate the experiments with the Namalwa F11 (transfected with the plasmid pCGMUdhfr) and the solid circles indicate those of the wild-type Namalwa cells.

Fig. 8 represents the enhancement of the survival of human neutrophils cultured in the presence of the supernatant of the culture media of the. Namalwa F11 and the wild-type Namalwa cells. The number on the horizontal line indicates the rate of dilution of the added supernatant. The open circles indicate the experiments with the Namalwa F11 and solid circles indicate the wild-type cells.

Fig. 9 shows the result of the SDS polyacrylamide gel electrophresis analysis of the hGM-CSF obtained in the following example.

## EXAMPLES

The present invention will be more precisely described by the following examples and they are not intended to limit the scope of the present invention.

It is contemplated that any usual modifications or improvements thereof are included in the scope of the present invention.

The activity of hGM-CSF was measured by the following three methods:

(a) Colony formation of human myeloid leukemia cell line KG-1 (A.J.Lusis et al., Proc.Nat.Acad.Sci., USA, 77:5346 (1980))

A mixture of 0.2ml of a sample and 0.8ml of 2% FCS α-MEM containing 20% FCS, 1.2% methylcellulose and $1 \times 10^3$ of KG-1 cells is incubated in a plastic Petri dish (35mm in diameter) for 14 days at 37°C under an atmosphere of saturated water vapor containing 5% $CO_2$. After that, the number of the colonies which consist of 50 or more cells is counted with a microscope.

(b) Human neutrophil survival assay (C.G.Begley et al., Blood, 68:162 (1986))

Human peripheral blood (5ml) is overlayed on Mono-Poly Resolving Medium (Flow Laboratories) (3.5ml) and centrifuged for 30 minutes at $300 \times g$ to prepare neutrophils.

To a Terasaki plate is placed $2 \times 10^2$ of the neutrophils in 10μl of 10% FCS/IMDM medium and 5μl of a sample added, which is then incubated for 22 hours at 37°C under an atmosphere of saturated water vapor containing 5% $CO_2$.

Although morphologic change of the neutrophils is observed when incubated in the absence of hGM-CSF, the neutrophils are morphologically changed with the enhancement of the survival when incubated in the presence of hGM-CSF.

The dose dependency is determined with the following scale:

(+): significant morphologic change;

(±): medium change;

(-): no change.

(c) Colony formation of hamster bone marrow cells

Bone marrow cells are isolated from femurs of a golden Syrian hamster under a sterile condition and suspended in 2% FCS/α-MEM. A mixture (1ml) of hGM-CSF sample to be tested, and $0.8 \times 10^4$ of the bone marrow cells in α-MEM medium containing 20% FCS and 0.8% methylcellulose 4000 is placed in a plastic Petri dish (35mm in diameter) and incubated for 7 days at 37°C under an atmosphere of saturated water vapor containing 5% $CO_2$. Then, the number of the colonies which consist of 50 or more cells is counted with a microscope. The number of the colonies increases in proportion to the concentration of the added hGM-CSF until it reaches a plateau level and the amount of hGM-CSF required to form half the maximum number of colonies is defined to be 50 units.

Example 1

Preparation of cDNA library and screening for hGM-CSF gene:-

Human leukemia cells line U937 (ATCC CRL1593) was cultured in the presence of $10^{-5}$M dexamethason and $10^{-7}$ phorbol myristate acetate, and centrifuged to give $1.2 \times 10^9$ cells. The cells were suspended in 48ml of a lysis buffer (0.2M Tris-HCL (pH8.0), 0.1M LiCl, 25mM EDTA, 1% sodium dodecyl sulfate (SDS), 5mM vanadyl ribonucleoside complex (Bethesda Research Labs.)). After 25ml of phenol was added, the suspension was stirred for 5 minutes, to which 24ml of chloroform was added.

The mixture was then stirred for 10 minutes and centrifuged to isolate an aqueous layer. After being washed twice with a mixture of phenol and chloroform (1:1) and twice with chloroform, the aqueous layer was subjected to the ethanol precipitation to give RNA, which was again subjected to the ethanol precipitation and suspended in 300μl of sterile water. The resulting RNA suspension was stored at -20°C. Polyadenylated RNA (poly(A)-RNA) was isolated from the RNA obtained as above by the method of Maniatis et al. with an oligo(dT)-cellulose column (Molecular Cloning, A Laboratory Manual, p.197, Cold Spring Harbor Lab., (1982)). A cDNA library was prepared using 20μg of the poly(A)-RNA.

After being diluted to 500μg/ml with distilled water, the poly(A)-RNA was heated for 3 minutes at 65°C and rapidly chilled in dry ice-propanol.

The poly(A)-RNA was added to a reaction solution (0.1M Tris-HCl (pH8.3), 0.01M $MgCl_2$, 0.01M DTT, 1mM dCTP, 1mM dGTP, 1mM dTTP, 0.5mM dATP, 100μCiα-$^{32}$p-dATP (3000 Ci/mmole) (Amersham)), 20μg oligo(dT)$_{12-18}$ (PL Biochem), 0.03M β-mercaptoethanol, 5mM vanadyl ribonucleoside complex

5

(Bethesda Research Labs.), 169 units of avian myeloblastosis virus (AMV) reverse transcriptase (Seikagaku Kogyo) to make the total volume of 200µl.

The mixture was allowed to react for 2 minutes at room temperature and for additional 6 hours at 44°C. The reaction was stopped by addition of 20µl of 0.5M EDTA (pH8.0). To the reaction mixture was added NaOH to 0.15M and the mixture was incubated for 12 hours at 37°C to hydrolyze the RNA. After neutralization by addition of 20µl of 1M Tris-HCl pH8.0, the mixture was extracted with phenol,chloroform saturated with TE buffer (10mM Tris-HCl (pH7.0), 1mM EDTA). The aqueous layer was collected and subjected to Sephadex G 150 gel filtration and single stranded cDNA (1µg) was recovered by ethanol precipitation.

The single stranded cDNA was dissolved in 200µl of a reaction solution (0.1M HEPES (pH 6.9), 0.01M $MgCl_2$, 2.5mM DTT, 0.07M KCl, 1mM dNTPs, 75 units of DNA polymerase I Klenow fragment (Boehringer Mannheim) and incubated for 21 hours at 14°C to yield double stranded cDNA. The reaction was stopped by addition of EDTA to final concentration of 0.0125M. The mixture was extracted with phenol/chloroform. The aqueous layer was collected and subjected to Sephadex G 150 gel filtration and double stranded cDNA was recovered by ethanol precipitation.

To complete the synthesis of double stranded cDNA, the recovered DNA was further incubated for 1 hour at 37°C in 50µl of a reaction solution (0.1M Tris-HCl (pH8.3), 0.01M $MgCl_2$, 0.01M DTT, 0.1M KCl, 1mM dNTPs, 0.03M β-mercaptoethanol, 42 units of AMV reverse transcriptase). After the reaction, the mixture was treated in the same manner as above. To remove the hairpin-loops, the double stranded cDNA was incubated at room temperature for 30 minutes in 50µl of a reaction solution (0.03M sodium acetate (pH4.5), 0.3M NaCl, 0.003M $ZnCl_2$, 100 units of S1 nuclease (Sigma)). The reaction mixture was treated in the same manner as described above to recover 900ng of the double stranded cDNA. The double stranded cDNA was again incubated for 90 minutes at 14°C in 60µl of a reaction solution (0.01M Tris-HCl (pH7.4), 0.01M $MgCl_2$, 1mM DTT, 0.05M NaCl, 80µM dNTPs, 12.5 units of DNA polymerase I Klenow fragment) to assure blunt ends of the cDNA. The cDNA was recovered by extraction with phenol/chloroform followed by Sephadex G 150 gel filtration.

The cDNA was then methylated with EcoRI methylase by incubating the cDNA for 20 minutes at 37°C in 30µl of a reaction solution (0.01M Tris-HCl (pH8.0), 0.01M EDTA, 24µg BSA, 0.005M DTT, 30µM S-adenosyl methionine, 5 units EcoRI methylase). The reaction was stopped by heating the mixture for 10 minutes at 70°C. The cDNA was recovered by extraction with phenol/chloroform followed by gel filtration. EcoRI linker was ligated to the cDNA at 14°C for 32 hours in 7.5µl of a reaction solution (0.05M Tris-HCl (pH7.8), 0.01M $MgCl_2$, 0.02M DTT, 1mM ATP, 50µg/ml BSA, 0.05µg phosphorylated EcoRI linker (New England Biolabs.), 300 units of T4 DNA ligase (New England Biolabs.)). The reaction mixture was then adjusted to 0.1M Tris-HCl (pH7.5), 0.05M NaCl, 5mM $MgCl_2$, 100µg/ml BSA. After addition of 125 units of EcoRI (New England Biolabs.), the mixture was incubated for 2 hours at 37°C. The cDNA was recovered by extraction with phenol/chloroform followed by gel filtration. The cDNA was dissolved in 100µl of a solution (0.01M Tris-HCl (pH7.5), 0.1M NaCl, 1mM EDTA) and fractionated on Biogel A50 (Bio-Rad). The cDNA longer than 500bp was collected to give 126ng of cDNA with the average size of 1200bp. The cDNA (20ng) was incubated with 5µg of EcoRI digested λgt10 for 15 minutes at 42°C in T4 DNA ligase buffer. After 2400 units of T4 DNA ligase and ATP (1mM final concentration) was added, the total volume of the mixture was adjusted to 50µl and allowed to react overnight at 14°C. Packaging was conducted with Amersham packaging mix (Amersham). The reaction mixture was diluted with 0.5ml of SM buffer (100mM NaCl, 10mM $MgSO_4$, 50mM Tris-HCl (pH7.5), 0.01% gelatin) and used for infection of E.coli C600 (ATCC 23724) to give $2 \times 10^{13}$ PFU/ml of cDNA library by the method of Maniatis et al. (Molecular Cloning, A Laboratory Manual, p.247, Cold Spring Harbor Lab. (1982))

As a probe for screening of the library, mouse GM-CSF cDNA was prepared as follows:

A cDNA library was prepared from EL-4 mouse thymoma cells (ATCC TIB39) in the same manner as in preparation of human cDNA library. Two DNAs with the following sequences were prepared as probes, based on the sequence of mouse GM-CSF cDNA (N.M Gough et al., Nature, 309:763 (1984)):

GMCSF-1: 5′ -CCAACTCCGGAAACGGACTG-3′
GMCSF-2: 5′ -CTTAAAACCTTTCTGACTG-3′

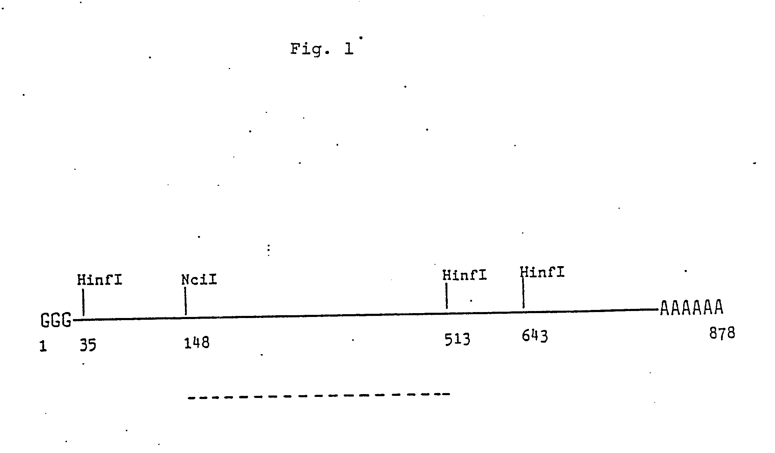

By screening the mouse cDNA library with these probes, mouse GM-CSF cDNA was cloned (see Fig. 1).

A DNA fragment of 365bp containing almost the complete coding region of mouse GM-CSF was prepared by digestion with restriction enzymes NciI and HinfI followed by purification in 5% polyacrylamide gel electrophoresis. This fragment was radio-labeled by nick-translation according to the method of Maniatis et al. (Molecular Cloning, A Laboratory Manual, p.109, Cold Spring Harbor Lab. (1982)).

The λgt10 phage particles were transferred onto nitrocellulose filters, and the filters were then incubated on LB plates containing 10mM MgSO₄ for 5 hours at 37°C. These filters were immersed in 0.5N NaOH for 5 minutes and then in 1M Tris-HCl (pH7.0) for 5 minutes. The phage DNA was fixed on the filters by baking at 80°C for 2 hours in vacuo. The fixed phage DNA was then hybridized to the radio-labeled probe DNA prepared from mouse GM-CSF cDNA.

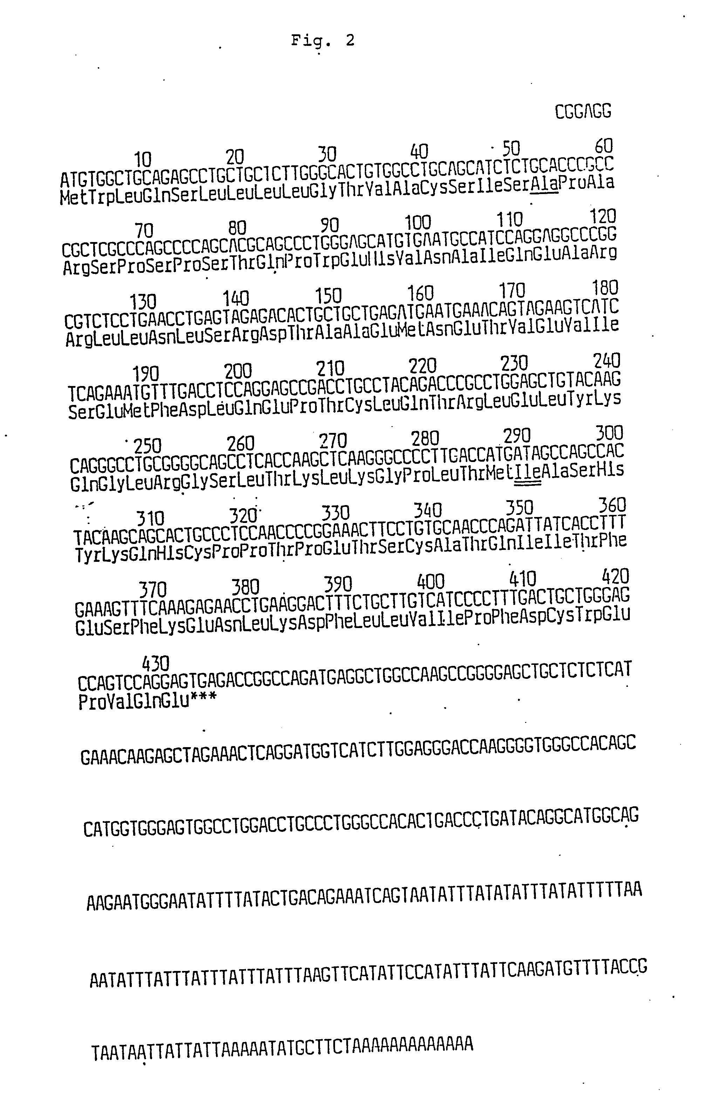

The nitrocellulose filters were placed in a hybridization buffer (6 × SSC, 0.2% polyvinyl pyrrolidone (M.W. 40,000), 0.2% Ficoll (M.W. 40,000), 0.2% bovine serum albumin, 0.05M Tris-HCl (pH7.5), 1M NaCl, 0.1% sodium pyrophosphate, 0.1% SDS, 10% dextran sulfate (M.W. 500,000), 100μg/ml denatured salmon sperm DNA) and the ³²P labeled probe DNA was added to the mixture, which was kept overnight at 65°C. The filters were washed with 2 × SSC containing 0.1% SDS at 65°C. After air-dried, the filters were autoradiographed. Screening of 1 × 10⁶ phages gave 20 positive plaques. The analysis of the clones revealed that one of the clones contained 767bp cDNA with an open reading frame for a protein consisting of 144 amino acids, which is identical over the coding region to that of the GM-CSF cDNA derived from Mo cells (G.G.Wong et al., Science, 228:810 (1985)) except the 297th nucleotide. The nucleotide sequence is shown in Fig. 2. The 297th nucleotide of the cDNA obtained as described above is G and the codon is for isoleucine, while the corresponding nucleotide of Mo cell GM-CSF DNA is A and the codon is for methionine.

The cDNA (2μg) was digested with EcoRI and incubated with 20 units of E.coli alkaline phosphatase for 1 hour at 65°C. After extraction with phenol/chloroform and ethanol precipitation, the resulting DNA was ligated to 1μg of pUC9 digested with EcoRI by incubation with 50 units of T4 DNA ligase for 3 hours at 15°C. The reaction mixture was used to transform E.coli JM83 (ATCC 35607).

From ampicillin resistant and β-galactosidase non-producing colonies, plasmid DNAs were isolated and insertion of GM-CSF cDNA in EcoRI site of pUC9 was confirmed by digestion with EcoRI. The plasmid was named as pU2B1c.

## Example 2

Construction of expression vectors:-

pU2B1c (8μg) was digested with 10 units of EcoRI and NcoI. DNA was recovered from the reaction mixture by extraction with phenol/chloroform and ethanol precipitation and incubated for 30 minutes at 37°C in 50μl of a reaction solution consisting of 1mM dNTPs, 0.1mM EDTA, 20mM NaCl, 7mM MgCl₂, 7mM Tris-HCl (pH7.5) and 8 units of DNA polymerase Klenow fragment to convert the sticky ends to blunt ends. The DNA was recovered by extraction with phenol/chloroform and ethanol precipitation. 3μg of BamHI linker (Takara) was ligated to the DNA in 20μl of a reaction solution (50mM Tris-HCl (pH7.5), 10mM MgCl₂, 10mM dithiothreitol 1mM ATP, 250 units T4 DNA ligase) for 3 hours at 15°C.

```
BamHI linker:  5'  pCGGATCCG

                       GCCTAGGCp  5'
```

After being recovered by extraction with phenol/chloroform and ethanol precipitation, the DNA was digested with 10 units of BamHI and subjected to preparative 5% polyacrylamide gel electrophoresis. The band corresponding to the GM-CSF cDNA fragment having BamHI sites at the ends was recovered from the gel by extraction with a buffer containing 0.5M ammonium acetate, 1mM EDTA pH8.0 followed by ethanol precipitation.

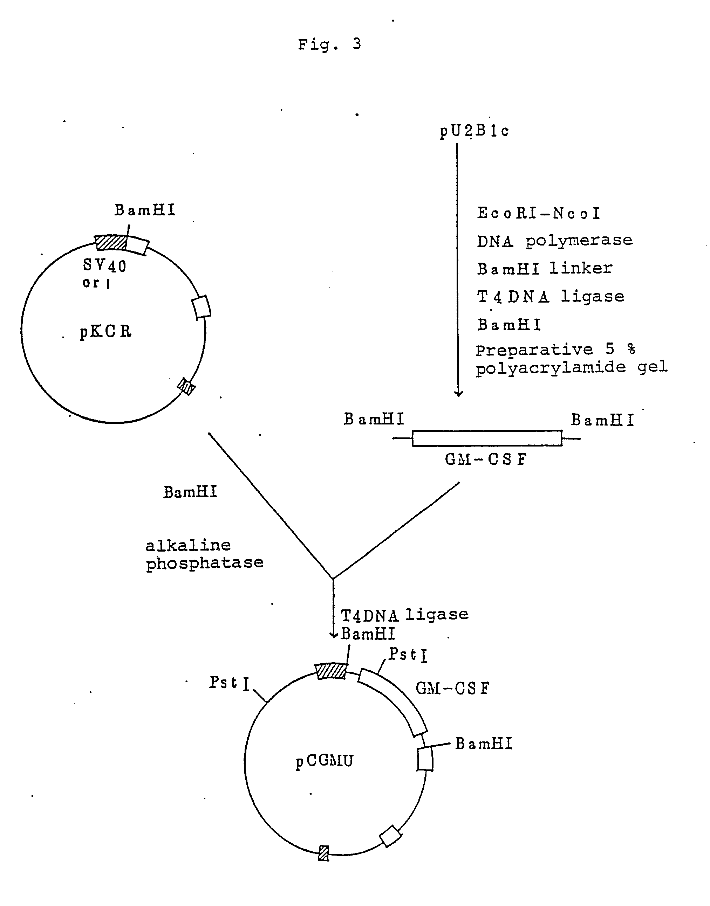

The resulting hGM-CSF cDNA fragment was ligated to 2μg of pKCR, which was digested with 10 units of BamHI, treated with 20 units of E.coli alkaline phosphatase for 1 hour at 65°C, and recovered by extraction with phenol/chloroform and ethanol precipitation. The reaction was carried out with 250 units of T4 DNA ligase for 3 hours at 15°C. The reaction mixture was used to transform E.coli DH1 (ATCC 33849). The plasmid DNAs derived from ampicillin resistant transformants were analyzed by BamHI digestion for insertion of hGM-CSF cDNA and by PstI digestion for determination of the orientation of the insert. The construction of the expression vector containing hGM-CSF cDNA was thus confirmed and the vector was named as pCGMU (Fig. 3).

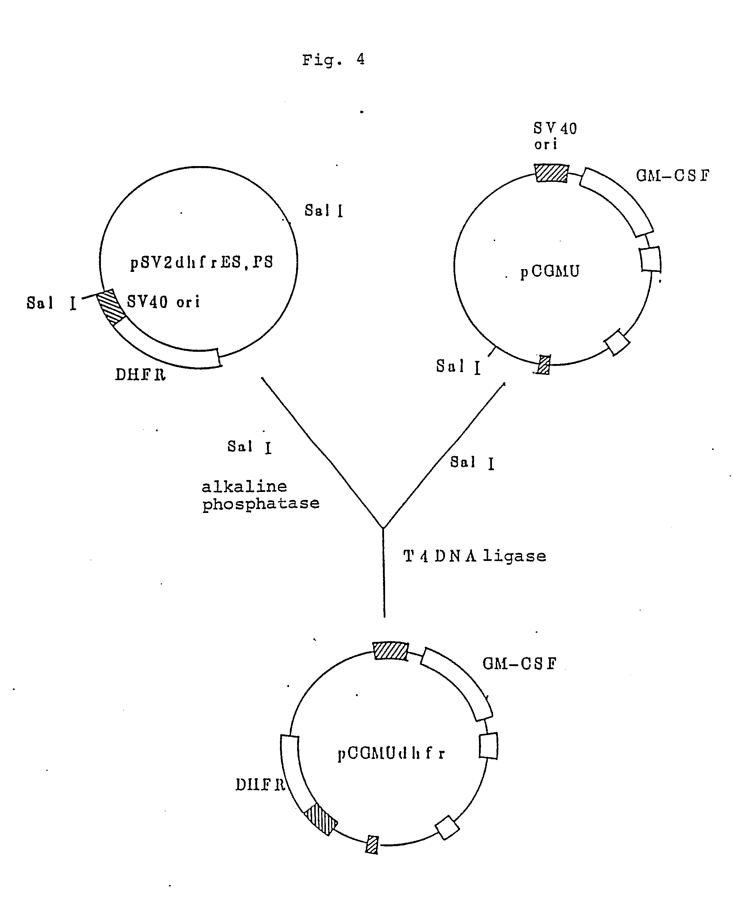

The plasmid pCGMU (2μg) was digested with 10 units of SalI. On the other hand, the plasmid pSV2dhfrES-PS (2μg), which was prepared by converting the EcoRI and PvuII sites of the plasmid pSV2dhfr (ATCC 37146) to SalI sites, was digested with 10 units of SalI and treated with E.coli alkaline phosphatase for 1 hour at 65°C. The both digested products were ligated with T4 DNA ligase for 3 hours at 15°C. The resulting reaction mixture was used to transform E.coli DH1 (ATCC 33849). The plasmid DNAs were isolated from the transformants and analyzed by digestion with various restriction enzymes including SalI to confirm the construction of the plasmid pCGMdhfr, the structure of which is shown in Fig. 4. In addition to the plasmid pCGMdhfr, the plasmid in which the dhfr gene was inserted in the reversed direction to the GM-CSF gene was also isolated and named as pCGMUdhfrRV.

Example 3

Transient expression in Namalwa cells:-

Namalwa cells ($5 \times 10^6$ cells) were suspended in 500$\mu$l of TBS buffer (25mM Tris-HCl (pH7.4), 137mM NaCl, 5mM KCl, 0.7mM $CaCl_2$, 0.5mM $MgCl_2$, 0.6mM $Na_2HPO_4$) containing 2.5$\mu$g of pCGMU and 1mg/ml of DEAE-dextran and allowed to stand for 30 minutes at room temperature. The suspension was centrifuged for 5 minutes at 500$\times$g. The pellets were resuspended in 1ml of 10% FCS/RPMI1640 containing 200$\mu$g/ml chloroquine and incubated for 4 hours at 37°C under an atmosphere of saturated water vapor containing 5% $CO_2$. After centrifugation for 5 minutes at 500$\times$g, the pellets of Namalwa cells were washed with TBS buffer and suspended in 1ml of 10% FCS/RPMI1640, which was incubated for 3 days at 37°C under an atmosphere of saturated water vapor containing 5% $CO_2$.

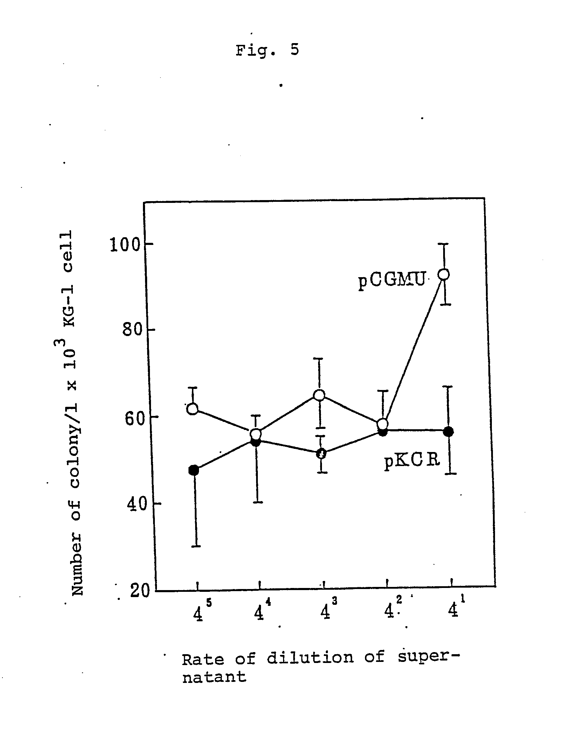

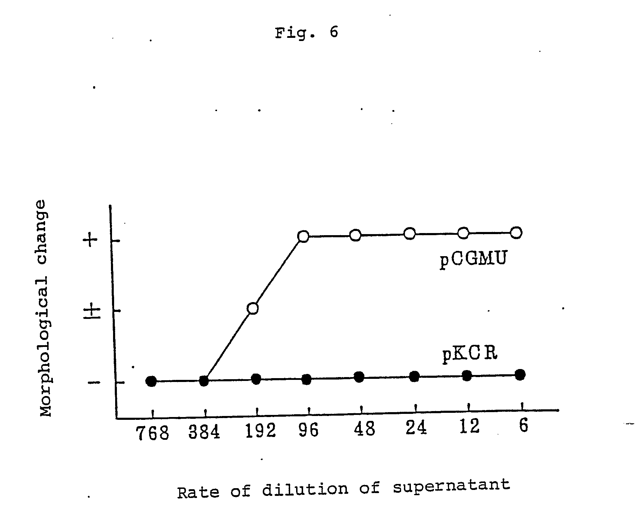

The hGM-CSF activity of the culture medium of the Namalwa cells transfected with pCGMU was examined by the colony formation method using KG-1 cells, of which results are shown in Fig. 5. The rate of colony formation of the supernatant of the culture medium of the Namalwa cells transfected with the plasmid pCGMU was higher than that of the Namalwa cells transfected with the plasmid pKCR. The hGM-CSF activity of the culture medium of the Namalwa cells was also examined by the human neutrophil survival assay. The results are shown in Fig. 6. The supernatant of the culture medium of Namalwa cells transfected with pKCR showed no hGM-CSF activity when 6-fold diluted. In contrast, the supernatant of the culture medium of the Namalwa cells transfected with pCGMU showed the activity even when it was 96-fold diluted.

Example 4

Stable expression in Namalwa cells:-

Namalwa cells ($10^7$ cells) were suspended in 1ml of phosphate buffered saline (137mM NaCl, 2.7Mm KCl, 8mM $Na_2HPO_4$, 1.5mM $KH_2PO_4$ (pH7.2)). After 75$\mu$g of PCGMUdhfr and 25$\mu$g of pSV2neo were added, the cell suspension was allowed to stand for 10 minutes at 4°C and placed in a vessel equipped with electrodes. A high voltage pulse (4kv/cm) was applied through the electrodes and then the suspension was centrifuged for 5 minutes at 500$\times$g. The pellets were suspended in 1ml of 10% FCS/RPMI1640 and incubated for 3 days at 37°C under an atmosphere of saturated water vapor containing 5% $CO_2$. After the incubation, the cells were spread onto 96-well tissue culture plates at a density of $2 \times 10^3$ cells/well and cultured in 10% FCS/RPMI1640 containing 200$\mu$g/ml of G-418. A half of the culture medium (100$\mu$l) was replaced by a fresh medium containing 400$\mu$g/ml of G-418 every 3 days, thereby the concentration of G-418 in the culture medium gradually increased. After 3 weeks of cultivation, colonies were formed. The hGM-CSF activity of each of the wells was measured by the human neutrophil survival assay to isolate the Namalwa cells acquiring the ability to produce hGM-CSF. The hGM-CSF-producing Namalwa cell line was named as Namalwa F11.

The Namalwa F11 cells were inoculated in 75cm$^2$ flask with 10% FCS/RPMI1640 containing 400$\mu$g of G-418 and incubated for 3 days at 37°C under an atmosphere of saturated water vapor containing 5% $CO_2$.

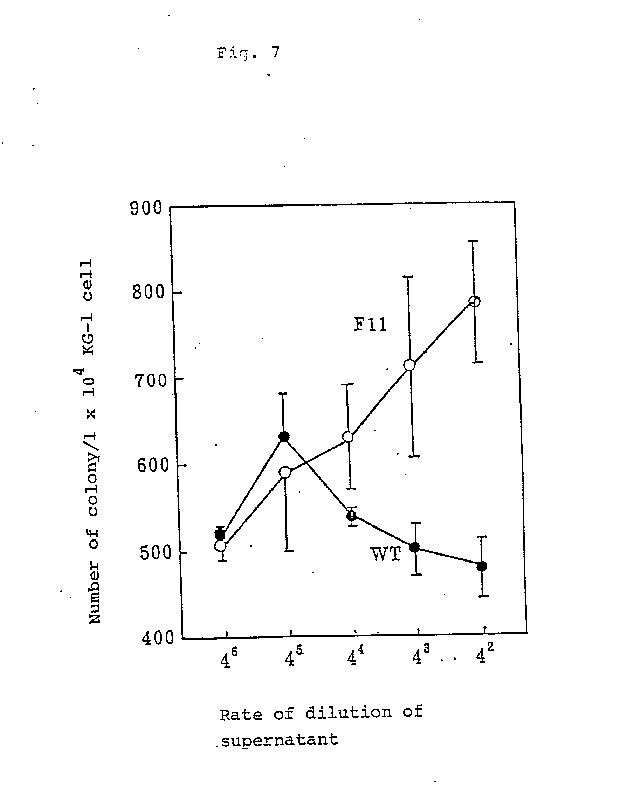

The hGM-CSF activity of the supernatant of the culture medium of Namalwa F11 was compared with that of the wild-type Namalwa cells by the colony formation method using KG-1 cells and the results are shown in Fig. 7. The Namalwa F11 showed higher activity than the wild cells.

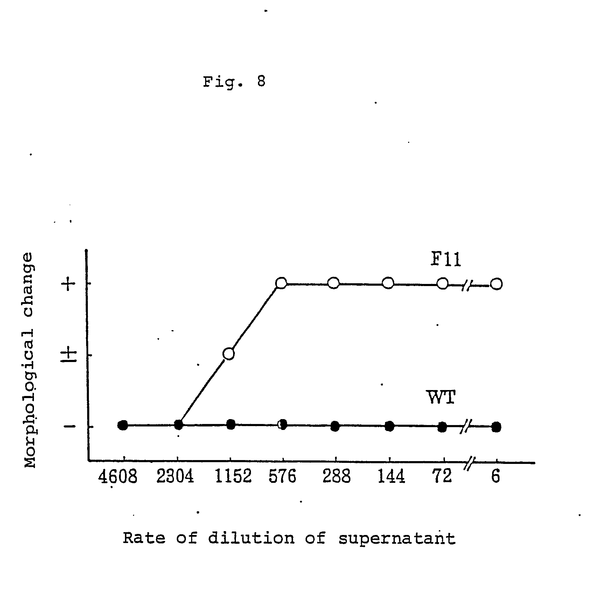

The hGM-CSF activity was also measured by human neutrophil survival assay method, the results are shown in Fig. 8.

The supernatant of the culture medium of the wild-type cells showed no activity when 6-fold diluted, while 576-fold diluted supernatant of the Namalwa F11 culture medium showed the activity. Similar results were obtained when the Namalwa cells were transfected with pCGMUdhfrRV. Thus, production of hGM-CSF was found in the Namalwa cells transfected with either pCGMU, pCGMUdhfr or pCGMUdhfrRV.

## Example 5

Preparation of rabbit anti-hGM-CSF antibody and an immunoabsorbent column:-

A male rabbit (New Zealand white) was immunized by intradermic injection of 100µg of E.coli-derived recombinant hGM-CSF, which was purified as described by A.W. Burgess et al. (Blood, 69:43 (1987)), together with Freund's complete adjuvant (Methods in Enzymology, 73:46, Academic Press, New York (1981)). 15.7ml of the serum (1ml of the serum neutralized $1.5-3.8 \times 10^5$ units of hGM-CSF) collected from the rabbit 2 to 3.5 months after immunization was applied to a Protein A-Sepahrose (Pharmacia Chemicals) column which was previously equilibrated with 0.15M NaCl/10mM sodium phosphate buffer (pH8.2). After washing with the same buffer, antibody was eluted with 1M NaCl/80mM glycine-HCl (pH2). The IgG fraction (28mg) was dialyzed against 10mM phosphate buffered saline (PBS) and passed through a PD-10 column equilibrated with 0.1M HEPES-NaOH (pH7.5) to substitute the PBS with this buffer. 2.5ml of Affi-Gel 10 (Bio-Rad) was washed on a glass filter with ice-chilled isopropanol followed by water. The gels were mixed with the antibody (17.5mg/9ml) prepared as above and stirred overnight for linking reaction. After the gels were recovered by filtration on a glass filter and resuspended in 9ml of 0.1M HEPES-NaOH (pH7.5), 0.25ml of 1M monoethanolamine-HCl (pH7.8) was added to the suspension and stirred for 1 hour at room temperature to prepare antibody-linked gels. The gels were washed on a glass filter with 4.5M MgCl₂/25mM Tris-HCl (pH7.5) (or 0.1M sodium citrate (pH2.5)) followed by PBS, and were then packed in a column to give an immunoabsorbent column.

## Example 6

Purification of hGM-CSF with an immunoabsorbent column:-

The hGM-CSF producing Namalwa cell line (Namalwa F11) was cultured in serum-free medium, Nutricyte H (Boehringer), and the supernatant (hGM-CSF activity: $8 \times 10^7$ units) was applied onto the immunoabsorbent column equilibrated with PBS at a flow rate of 10ml/hr. The column was washed with 250ml of 1M NaCl/10mM Tris-HCl (pH7.5) and eluted with 20ml of 4.5M MgCl₂/25mM Tris-HCl (pH7.5) at a flow rate of 10ml/hr to give a solution containing highly purified hGM-CSF ($4.4 \times 10^7$ units; recovery, 55%). In this experiment, hGM-CSF activity was measured by the method of colony formation of hamster bone marrow cells. The amino acid sequence of this hGM-CSF was analysed with a gas-phase protein sequencer (Applied Biosystems Inc.) and the sequence of the first twenty amino acids of the N-terminal region was found as follows:
Ala-Pro-Ala-Arg-Ser-Pro-X-Pro-X-Thr-Gln-Pro-Trp-Glu-His-Val-Asn-Ala-Ile-Gln-(X, unidentified)

The above sequence was in agreement with the corresponding part of the deduced amino acid sequence shown in Fig. 2, which indicates that the hGM-CSF obtained as above was highly homogeneous. The serine residues at positions 7 and 9 were not detected. This is a reasonable result because it has been known that these residues of hGM-CSF are the sites linking mucin-type sugar chains (K. Kaushansky et al., Biochemistry, 26:4861 (1987)).

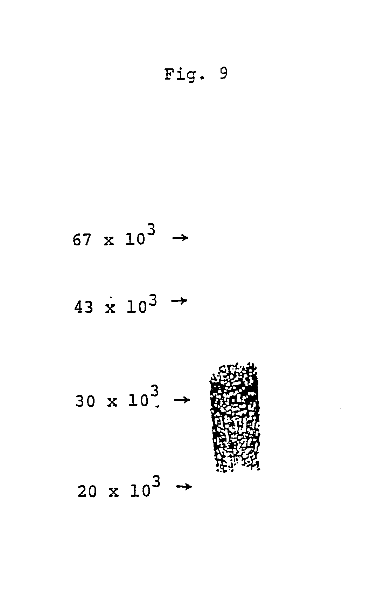

SDS-Polyacrylamide gel electrophoresis analysis of the hGM-CSF fraction eluted from the immunoabsorbent column indicated that the hGM-CSF produced in Namalwa cells has microheterogeneity with apparent molecular weights of 22,000 to 36,000 as shown in Fig. 9. This is likely due to the heterogeneity of the sugar chains of the hGM-CSF as reported elsewhere (K.Kaushansky et al., Biochemistry, 26:4861 (1987); P.Moonen et al., Pro.Nat.Acid.Sci., USA, 84:4428 (1987); R.E. Donahue et al., Cold Spring Harbor Symposia on Quantitative Biology, 51:685 (1986)).

## Claims

1. Human granulocyte-macrophage colony stimulating factor produced in human cells transfected with an exogenous DNA encoding the said factor.

2. Transformed human cells capable of expressing an exogenous gene encoding human granulocyte-macrophage colony stimulating factor and their progeny.

3. Transformed human cells and their progeny according to Claim 2, which is progeny of Namalwa cells.

4. Transformed human cells according to Claim 2 or 3, which is transfected with the plasmid selected from the group consisting of plasmid pCGMU, plasmid pCGMUdhfr and plasmid pCGMUdhfrRV and their progeny.

5. A process for producing human granulocyte-macrophage colony stimulating factor which comprises culturing the human cells transfected with a DNA coding for the said factor or their progeny and recovering it.

6. A process according to Claim 5, wherein the human cells are Namalwa cells.

7. Transformed human cells according to Claim 5 or 6, wherein the said human cells are the human cells transfected with the plasmid selected from the group consisting of plasmid pCGMU, plasmid pCGMUdhfr and plasmid pCGMUdhfrRV or their progeny.

8. A plasmid selected from the group consisting of plasmid pCGMU, plasmid pCGMUdhfr and plasmid pCGMUdhfrRV.

9. A microorganism harboring a plasmid selected from the group consisting of plasmid pCGMU, plasmid pCGMUdhfr and plasmid pCGMUdhfrRV.

10. A microorganism according to Claim 9, which belongs to E.coli.

Fig. 1

## Fig. 2

CGGAGG

```
          10        20        30        40       ·50        60
ATGTGGCTGCAGAGCCTGCTGCTCTTGGGCACTGTGGCCTGCAGCATCTCTGCACCCGCC
MetTrpLeuGlnSerLeuLeuLeuLeuGlyThrValAlaCysSerIleSerAlaProAla

          70        80        90       100       110       120
CGCTCGCCCAGCCCCAGCACGCAGCCCTGGGAGCATGTGAATGCCATCCAGGAGGCCCGG
ArgSerProSerProSerThrGlnProTrpGluIleValAsnAlaIleGlnGluAlaArg

         130       140       150       160       170       180
CGTCTCCTGAACCTGAGTAGAGACACTGCTGCTGAGATGAATGAACAGTAGAAGTCATC
ArgLeuLeuAsnLeuSerArgAspThrAlaAlaGluMetAsnGluThrValGluValIle

         190       200       210       220       230       240
TCAGAAATGTTTGACCTCCAGGAGCCGACCTGCCTACAGACCCGCCTGGAGCTGTACAAG
SerGluMetPheAspLeuGlnGluProThrCysLeuGlnThrArgLeuGluLeuTyrLys

        ·250       260       270       280       290       300
CAGGGCCTGCGGGGCAGCCTCACCAAGCTCAAGGGCCCCTTGACCATGATAGCCAGCCAC
GlnGlyLeuArgGlySerLeuThrLysLeuLysGlyProLeuThrMetIleAlaSerHis

         310       320       330       340       350       360
TACAAGCAGCACTGCCCTCCAACCCCGGAAACTTCCTGTGCAACCCAGATTATCACCTTT
TyrLysGlnHisCysProProThrProGluThrSerCysAlaThrGlnIleIleThrPhe

         370       380       390       400       410       420
GAAAGTTTCAAAGAGAACCTGAAGGACTTTCTGCTTGTCATCCCCTTTGACTGCTGGGAG
GluSerPheLysGluAsnLeuLysAspPheLeuLeuValIleProPheAspCysTrpGlu

         430
CCAGTCCAGGAGTGAGACCGGCCAGATGAGGCTGGCCAAGCCGGGGAGCTGCTCTCTCAT
ProValGlnGlu***

GAAACAAGAGCTAGAAACTCAGGATGGTCATCTTGGAGGGACCAAGCGGGTGGGCCACAGC

CATGGTGGGAGTGGCCTGGACCTGCCCTGGGCCACACTGACCCTGATACAGGCATGGCAG

AAGAATGGGAATATTTTATACTGACAGAAATCAGTAATATTTATATATTTATATTTTTAA

AATATTTATTTATTTATTTATTTAAGTTCATATTCCATATTTATTCAAGATGTTTTACCG

TAATAATTATTATTAAAAAATATGCTTCTAAAAAAAAAAAAAA
```

Fig. 3

Fig. 4

Fig. 5

Rate of dilution of super-
natant

Fig. 6

pCGMU

pKCR

Rate of dilution of supernatant

Fig. 7

Rate of dilution of
supernatant

Fig. 8

Rate of dilution of supernatant

Fig. 9

$67 \times 10^3 \rightarrow$

$43 \times 10^3 \rightarrow$

$30 \times 10^3 \rightarrow$

$20 \times 10^3 \rightarrow$

| | | DOCUMENTS CONSIDERED TO BE RELEVANT | | EP 88103067.0 |
|---|---|---|---|---|

| Category | Citation of document with indication. where appropriate, of relevant passages | Relevant to claim | CLASSIFICATION OF THE APPLICATION (Int. Cl 4) |
|---|---|---|---|
| P,X | WO - A1 - 87/02 060 (BIOGEN N.V.)  * Fig. 1; claims 9,12-14 * | 1 | C 12 N 15/00 |
| P,A | * Claims 8,13,14 * | 5 | C 12 P 21/00  C 12 N  5/00 |
| D,X | WO - A1 - 86/03 225 (SCHERING BIO-TECH CORPORATION)  * Fig. 1 * | 1 | C 12 N  1/20  /(C 12 N  1/20  C 12 R  1:19) |
| D,A | * Claim 1 * | 5 | |
| D,X | PROCEEDINGS OF THE NATIONAL ACADEMY OF SCIENCES OF THE UNITED STATES OF AMERICA, vol. 82, no. 13, July 1985, Baltimore, USA  F.LEE et al. "Isolation of cDNA for human granulocytemacrophage colony-stimulating factor by functional expression in mammalian cells" pages 4360-4364  * Page 4362; fig. 3 * | 1 | |
| D,A | * Abstract * | 5 | TECHNICAL FIELDS SEARCHED (Int. Cl 4)  C 12 N  C 12 P |
| P,X | EP - A2 - 0 228 018 (HOECHST AKTIENGESELLSCHAFT)  * Claim 1 * | 1 | |
| P,A | * Claims 1,2 * | 5 | |
| X | EP - A2 - 0 183 350 (IMMUNEX CORPORATION)  * Claim 5; fig. 2 * | 1 | |
| A | * Claim 13 * | 5 | |

The present search report has been drawn up for all claims

| Place of search | Date of completion of the search | Examiner |
|---|---|---|
| VIENNA | 19-05-1988 | WOLF |